# EUROPEAN PATENT SPECIFICATION

(11) **EP 1 423 112 B1**
(45) Date of publication and mention of the grant of the patent: **18.10.2006**
(21) Application number: 02760969.2
(22) Date of filing: 23.08.2002
(51) Int. Cl.: A61K 31/422, A61K 31/4045, A61K 31/55, A61P 25/06, A61P 25/18

(54) **A COMBINATION OF QUETIAPINE AND ZOLMITRIPTAN**
KOMBINATION AUS QUETIAPIN UND ZOLMITRIPTAN
COMBINAISON DE QUETIAPINE ET DE ZOLMITRIPTAN

(30) Priority: 27.08.2001 SE 0102855
(43) Date of publication of application: 02.06.2004
(73) Proprietor: AstraZeneca AB, 151 85 Södertälje (SE)
(72) Inventor: LEE, David, West Chester, PA 19382 (US)
(86) International application number: PCT/SE2002/001507
(87) International publication number: WO 2003/018009

(56) References cited:
- WO-A1-01/21179
- WO-A1-01/39772
- WO-A1-97/35584
- WO-A1-97/45124
- GB-A- 2 324 961
- DATABASE CAPLUS [Online] DOC. NO. 128:225529 CALEY CHARLES F. ET AL.: 'Focus on quetiapine: the fourth atypical antipsychotic', XP002957954 Retrieved from STN Database accession no. 1998:130310 & FORMULARY vol. 33, no. 2, 1998, pages 105 - 106, 109-110, 112, 115-116, 119
- MERCER A.J. ET AL.: 'Lack of an effect of zolmitriptan (Zomig, 311C90) on psychometric task performance: results of a placebo-controlled study in healthy volunteers' PSYCHOPHARMACOLOGY vol. 140, no. 4, 1998, pages 398 - 404, XP002957957
- CALEY ET AL: 'Focus on quetiapin' FORMULARY vol. 33, 1998, pages 105 - 119, XP001009557

## Description

The present invention relates to a combination comprising the antipsychotic dopamine D2/5-HT_{2A} agent quetiapine or a pharmaceutically acceptable salt thereof and the 5-HT_{1B/1D} agonist zolmitriptan or a pharmaceutically acceptable salt thereof, pharmaceutical compositions, processes for its preparation, and the use thereof in the manufacture of a medicament for the treatment of disease and more particularly for the treatment of diseases typically treated with 5-HT_{1B/1D} agonists and/or atypical antipsychotics. Such diseases are migraine and related disorders, in particularly, migraine or related conditions, and for reducing or eliminating of migraine recurrence.

The co-administration of quetiapine or a pharmaceutically acceptable salt thereof and zolmitriptan or a pharmaceutically acceptable salt thereof leads to benefits over existing therapies.

Quetiapine has the chemical name 11-[4-[2-(2-hydroxyethoxy)ethyl]-1-piperazinyl]dibenzo[b,f][1,4]thiazepine and is an antipsychotic agent that has been sold as the fumarate salt under the trademark Seroquel^{™} for a number of years. Quetiapine fumarate is marketed for the treatment of schizophrenia and related disease conditions. A considerable body of literature describes how to use quetiapine fumarate. Specific references for the preparation and use of this agent are EP 240228 and 282236, US 4,879,288 and WO 97/45124.

Zolmitriptan has the chemical name (S)-4-{{3-[2-(dimethylaminoethyl]-1H-indol-5-yl]methyl]-2-oxazolidinone. Specific references for the preparation and use of this agent are EP 486666 and WO 97/06162. Zolmitriptan is a selective 5-HT_{1B/1D}-receptor agonist. During migraine excessive cerebrovascular dilation and neurogenic inflammatory processes are considered to contribute to pain. The 5-HT_{1B/1D}-receptors mediate cerebrovascular vasoconstriction and inhibit neurogenic inflammation. 5-HT_{1B/1D} receptor agonists are beneficial in the treatment (including prophylaxis) of disease conditions wherein vasoconstriction and neurogenic inflammation in the cerbrovascular bed is indicated, for example migraine, cluster headache and headache associated with vascular disorders, hereinafter referred to collectively as migraine. Zolmitriptan has been developed for the acute treatment of migraine in the form of a 2.5 mg and 5 mg tablet intended to be taken up to a maximum of 15 mg per day.

Although zolmitriptan is a successful drug of considerable benefit to migraine sufferers, there is a continuing need for alternative methods for the direct treatment of migraine and the prophylactic treatment of migraine and the treatment (direct and prophylactic) of related conditions.

The co-administration of quetiapine or a pharmaceutically acceptable salt thereof and zolmitriptan or a pharmaceutically acceptable salt thereof is expected to provide benefit in the treatment of migraine and related conditions for example the symptomatic treatment of pain relief, decreasing nausea, decreasing photophobia and phonophobia.

For example benefits may include greater efficacy where response to zolmitriptan alone is not as good as expected; greater efficacy (for example reduction in frequency and/or severity of episodes) against migraine attacks and their symptoms for example against persistent headache where recurrence is a problem and the rate of relapse needs to be reduced or eliminated including for example as part of a withdrawal regimen; where a migraine patient also suffers from psychoses, depression or anxiety; and a further benefit could be that lower doses of zolmitriptan may be given leading to greater tolerability and/or safety. In summary, the benefits may include increased number of patients that exhibit efficacy to zolmitriptan, increased number of patients that achieve pain-free status or near pain-free status, and decreased frequency of headache recurrence. Accordingly, the present invention provides a combination comprising quetiapine or a pharmaceutically acceptable salt thereof and zolmitriptan or a pharmaceutically acceptable salt thereof.

Other objects of the present invention provide a pharmaceutical composition comprising said combination, processes for the preparation of said combinations, a kit comprising said combination, optionally with instructions for use, and the use of said combination for the manufacture of a medicament for the treatment of migraine and related disorders, and for reduction or elimination of migraine recurrence.

Quetiapine has demonstrated efficacy against agitation and anxiety; agitation and anxiety may contribute to the onset and persistence of migraine headaches leading to a further benefit of using quetiapine and zolmitriptan in combination.

Treating includes the direct and prophylactic treatment of migraine or related condition. Direct treatment includes elimination, reduction and alleviation of the disease and/or symptoms; prophylactic treatment includes preventative measures.

Migraine and related conditions include conditions wherein vasoconstriction in the carotid vascular bed is indicated; for example, migraine, cluster headache, headache associated with vascular disorders and aura.

Quetiapine or a pharmaceutically acceptable salt thereof and zolmitriptan or a pharmaceutically acceptable salt thereof may be administered in the same formulation (co-formulation) or separately, conveniently using marketed dosage forms and strengths.

The dosage form, dosage strength and frequency of dosing of zolmitriptan administered depends on various factors known in the art including the weight, age and sex of the patient being treated and the particular migraine disease condition being treated. Typically, a unit dose of 0.5 mg to 15 mg (for example, 0.5 mg, 1.0 mg, 2.5 mg, 5.0 mg and 10 mg) of zolmitriptan is administered to the patient in need thereof. Zolmitriptan may be administered orally, intravenously, intranasally or by a fast-melt formulation that rapidly dissolves in the mouth. Typically, a daily dose for an adult human is in the range of 0.5 mg to 15 mg per day depending on the route of administration and the particular needs of the patient.

Formulations of zolmitriptan may be prepared according to EP 486666, WO 01/39772, US 5,178,878, US 6,024,981 and according to methods generally known in the art of formulation technology.

The dosage form, dosage strength and frequency of dosing of quetiapine administered also depends on the various factors known in the art including the weight, age and sex of the patient being treated. Typically, a unit dose of 5 mg to 50 mg (for example, 5 mg, 10 mg, 25 mg and 40 mg) of quetiapine is administered to the patient in need thereof. Quetiapine may be administered parenterally or may be administered orally either in a conventional tablet or capsule or in a modified formulation such as a controlled, delayed or sustained release formulation.

Formulations of quetiapine may be prepared according to EP 240228 and according to methods generally known in the art of formulation technology. WO 01/21179 describes a formulation in the form of granules of quetiapine, which could be a further aspect of administration.

In a particular aspect, quetiapine or a pharmaceutically acceptable salt thereof is administered orally and zolmitriptan or a pharmaceutically acceptable salt thereof is administered orally or intranasally; preferably the drugs are administered orally, particularly the zolmitriptan is administered as a tablet or a fast melt formulation. Suitably zolmitriptan or a pharmaceutically acceptable salt thereof is administered in a 2.5 mg or 5 mg unit dose and quetiapine or a pharmaceutically acceptable salt thereof is administered in a 25 mg unit dose.

In another particular aspect, quetiapine or a pharmaceutically acceptable salt thereof is administered in a controlled, delayed or sustained release dosage form. For example, dosage forms according to WO 97/45124 may be used. Such dosage forms provide a generally uniform and constant rate of release over an extended period of time to achieve a stable, desired blood (plasma) level of quetiapine without the need for frequent administration. This is particularly beneficial if the patient suffers from recurring migraine attacks as a stable level of quetiapine can be maintained.

In a particular aspect, the amount of zolmitriptan administered to a patient in need thereof can be lower than the amount of zolmitriptan that would be required if zolmitriptan were administered in the absence of quetiapine. Lower amounts of zolmitriptan may avoid side effects and/or improve tolerability in certain patients. In another particular aspect, the amount of quetiapine administered to a patient in need thereof can be lower than the amount of quetiapine that would be required if quetiapine were administered in the absence of zolmitriptan. Lower amounts of quetiapine may avoid side effects and/or improve tolerability in certain patients.

In another particular aspect, the administration of zolmitriptan and quetiapine can lead to greater and/or longer efficacy than would be the case in the absence of quetiapine.

In another aspect, quetiapine or a pharmaceutically acceptable salt thereof and zolmitriptan or a pharmaceutically acceptable salt thereof may be formulated in the same pharmaceutical composition with pharmaceutically acceptable carriers. Such a pharmaceutical composition may be administered orally (e.g. tablets, capsules) or by injection (e.g. a solution). Such compositions may be prepared in a conventional manner with suitable pharmaceutically acceptable carriers such as binding agents, fillers, disintegrates and solubilizing agents.
There are currently no animal models of migraine that are considered to be completely predictive of efficacy in humans. However, animal models are available that are considered to mimic important aspects of migraine pathophysiology. The injection of lipopolysaccharides (LPS) into the cerebral ventricles of rats causes cerebral inflammation, an inflammatory process that may occur in migraine. Directing an air puff to the head of the rat and measuring ultrasonic vocalizations can assess the pain resulting from this process. This method is described in detail below:

### i) Administration of LPS

Rats are allowed to habituate in the experimental laboratory for 15-20 minutes prior to treatment. Cerebral inflammation is induced by administration of LPS (endotoxin of gram-negative *E*. *coli* bacteria serotype 0111:B4, Sigma). LPS (2,4 µg) is injected intracerebro-ventricularly (i.c.v.), in a volume of 10µl using standard stereotaxic surgical techniques under isoflurane anesthesia. The skin between the ears is pushed rostrally and a longitudinal incision of about 1cm is made to expose the skull surface. The puncture site is determined by the coordinates: 0.8 mm posterior to the bregma, 1.5 mm lateral (left) to the lambda (sagittal suture), and 5 mm below the surface of the skull (vertical) in the lateral ventricle. LPS is injected via a sterile stainless steel needle (26-G 3/8) of 5 mm long attached to a 100-µl Hamilton syringe by polyethylene tubing (PE20; 10-15 cm). A 4 mm stopper made from a cut needle (20-G) is placed over and secured to the 26-G needle by silicone glue to create the desired 5mm depth.

Following the injection of LPS, the needle remains in place for an additional 10 seconds to allow diffusion of LPS, then is removed. The incision is closed, and the rat is returned to its original cage and is allowed to rest for a minimum of 3.5 hours prior to testing.

### ii) Experimental setup for air-puff stimulation

The rats remain in the experimental laboratory following LPS injection and drug administration. At the time of testing all rats are removed and placed outside the laboratory. One rat at a time is brought into the testing laboratory and placed in a clear box (9 × 9 × 18 cm) which is then placed in a sound-attenuating ventilated cubicle measuring 62(w) ×35(d) ×46(h) cm (BRS/LVE, Div. Tech-Serv Inc). Air-puffs are delivered, through an air output nozzle of 0.32 cm, which is controlled by a system (AirStim®, San Diego Instruments) capable of delivering puffs of air of fixed duration (0.2 s) and fixed intensity with a frequency of 1 puff per 10 seconds. A maximum of 10 puffs are administered, or until vocalization starts, which ever comes first. The first air puff marks the start of recording.

### iii) Experimental setup for and ultrasound recording

The vocalizations are recorded for 10 minutes using microphones (G.R.A.S. sound and vibrations, Vedbaek, Denmark) placed inside each cubicle and controlled by LMS®(LMS® CADA-X 3.5B, Data Acquisition Monitor, Troy, Michigan) software. The frequencies between 0 and 32000Hz are recorded, saved and analyzed by the same software (LMS® CADA-X 3.5B, Time Data Processing Monitor and UPA (User Programming and Analysis)). The number of ultrasonic vocalizations induced by air puffs is taken as a measure of pain experienced by the rats.

### iv) Analysis

The recordings are run through a series of statistical and Fourier analyses to filter (between 20-24kHz) and to calculate the parameters of interest. The data are expressed as the mean ± SEM. Statistical significance are assessed using T-test for comparison between naive and LPS-treated rats, and one way ANOVA followed by Dunnett's multiple comparison test (post-hoc) for drug effectiveness. A difference between groups is considered significant with a minimum *p* value of ≤0.05. Experiments are repeated a minimum of two times.

### Examples

1) An adult patient experiencing the onset of migraine is treated with quetiapine fumarate (25 mg) and zolmitriptan (5 mg). Quetiapine fumarate is administered as a tablet ('Seroquel®') and zolmitriptan is also administered as a tablet ('Zomig®').
2) A tablet is prepared as follows:

| mg | |
|---|---|
| Quetiapine Fumarate | 28 |
| Zolmitriptan | 5 |
| Povidone | 7.00 |
| Calcium Hydrogen Phosphate | 8.72 |
| Microcrystalline cellulose | 28.50 |
| Lactose monohydrate | 19.00 |
| Sodium starch glycollate | 7.00 |
| Magnesium stearate | 1.00 |

| Coating | |
|---|---|
| Methylhydroxypropylcellulose | 1.56 |
| Macrogel 400 | 0.31 |
| Titanium dioxide | 0.59 |
| Ferric oxide, red | 0.02 |
| Ferric oxide, yellow | 0.02 |

The ingredients are mixed with purified water, blended and compressed into tablets, which are then coated.

## Claims

1. A combination comprising quetiapine or a pharmaceutically acceptable salt thereof and zolmitriptan or a pharmaceutically acceptable salt thereof.

2. A combination according to claim 1 for use simultaneously, sequentially or separately as a medicament.

3. A combination according to claim 1 for use simultaneously, sequentially or separately for the treatment of migraine or related conditions.

4. A combination according to claim 1 for use simultaneously, sequentially or separately for reducing or eliminating of migraine recurrence.

5. A combination according to anyone of claims 1-4 wherein quetiapine or a pharmaceutically acceptable salt thereof is to be administered orally and zolmitriptan or a pharmaceutically acceptable salt thereof is to be administered orally or intranasally.

6. A combination according to any one of claim 5 wherein quetiapine or a pharmaceutically acceptable salt thereof is to be administered orally and zolmitriptan or a pharmaceutically acceptable salt thereof is to be administered orally.

7. A combination according to claim 6 wherein quetiapine or a pharmaceutically acceptable salt thereof is to be administered as a tablet and zolmitriptan or a pharmaceutically acceptable salt thereof is to be administered as a tablet.

8. A combination according to anyone of claims 1-7 wherein zolmitriptan or a pharmaceutically acceptable salt thereof is to be administered as a fast melt formulation.

9. A combination according to any one of claims 1-7 wherein quetiapine or a pharmaceutically acceptable salt thereof is to be administered in a controlled delayed or sustained release dosage form.

10. A combination according to any one of claims 1-9 wherein zolmitriptan or a pharmaceutically acceptable salt thereof is to be administered in a unit dose of 0.5 to 15 mg and quetiapine or a pharmaceutically acceptable salt thereof is to be administered in a unit dose of 5 to 50 mg.

11. A combination according to claim 10 wherein zolmitriptan or a pharmaceutically acceptable salt thereof is to be administered in a 5 mg unit dose and quetiapine or a pharmaceutically acceptable salt thereof is to be administered in a 25 mg unit dose.

12. A combination according to any one of claims 1-11 wherein the quetiapine pharmaceutically acceptable salt is quetiapine fumarate.

13. A combination according to any one of claims 1-12 comprising zolmitriptan and quetiapine fumarate.

14. A pharmaceutical composition comprising the combination according to any one of claims 1-13 and optionally a pharmaceutical carrier or diluent.

15. A pharmaceutical composition according to claim 14 for use simultaneously, sequentially or separately as a medicament.

16. A pharmaceutical composition according to claim 15 for use simultaneously, sequentially or separately in the treatment of migraine or related conditions.

17. A combination comprising a first pharmaceutical composition comprising quetiapine or a pharmaceutically acceptable salt thereof and optionally a pharmaceutical carrier or diluent and a second pharmaceutical composition comprising zolmitriptan or a pharmaceutically acceptable salt thereof and optionally a pharmaceutical carrier or diluent.

18. Use of a combination according to any one of claims 1-17 for the manufacture of a medicament for administration simultaneously, sequentially or separately to a mammal for the treatment of migraine or related conditions.

19. Use of a combination according to any one of claims 1-17 for the manufacture of a medicament for administration simultaneously, sequentially or separately to a mammal for reducing or eliminating of migraine recurrence.

20. A process for the preparation of a combination according to any one of claims 1-16 wherein quetiapine or a pharmaceutically acceptable salt thereof and zolmitriptan or a pharmaceutically acceptable salt thereof are incorporated in the same pharmaceutical composition.

21. A process for the preparation of a combination according to any one of claims 1-17 wherein quetiapine or a pharmaceutically acceptable salt thereof and zolmitriptan or a pharmaceutically acceptable salt thereof are incorporated in different pharmaceutical compositions.

22. A kit comprising quetiapine or a pharmaceutically acceptable salt thereof and zolmitriptan or a pharmaceutically acceptable salt thereof, optionally with instructions and/or labeling for the use.

23. The kit according to claim 22 wherein quetiapine or a pharmaceutically acceptable salt thereof and zolmitriptan or a pharmaceutically acceptable salt thereof are for simultaneous or contemporaneous administration.

## Patentansprüche

1. Kombination, umfassend Quetiapin oder ein pharmazeutisch annehmbares Salz davon und Zolmitriptan oder ein pharmazeutisch annehmbares Salz davon.

2. Kombination nach Anspruch 1 zur simultanen, sequentiellen oder separaten Verwendung als Medikament.

3. Kombination nach Anspruch 1 zur simultanen, sequentiellen oder separaten Verwendung zur Behandlung von Migräne oder verwandten Erkrankungen.

4. Kombination nach Anspruch 1 zur simultanen, sequentiellen oder separaten Verwendung zur Verringerung oder Beseitigung des Wiederauftretens von Migräne.

5. Kombination nach einem der Ansprüche 1 bis 4, wobei Quetiapin oder ein pharmazeutisch annehmbares Salz davon oral verabreicht werden soll und Zolmitriptan oder ein pharmazeutisch annehmbares Salz davon oral oder intranasal verabreicht werden soll.

6. Kombination nach Anspruch 5, wobei Quetiapin oder ein pharmazeutisch annehmbares Salz davon oral verabreicht werden soll und Zolmitriptan oder ein pharmazeutisch annehmbares Salz davon oral verabreicht werden soll.

7. Kombination nach Anspruch 6, wobei Quetiapin oder ein pharmazeutisch annehmbares Salz davon als Tablette verabreicht werden soll und Zolmitriptan oder ein pharmazeutisch annehmbares Salz davon als Tablette verabreicht werden soll.

8. Kombination nach einem der Ansprüche 1 bis 7, wobei Zolmitriptan oder ein pharmazeutisch annehmbares Salz davon als sich rasch auflösende Formulierung verabreicht werden soll.

9. Kombination nach einem der Ansprüche 1 bis 7, wobei Quetiapin oder ein pharmazeutisch annehmbares Salz davon in einer Dosierform mit kontrollierter verzögerter kontinuierlicher Freisetzung verabreicht werden soll.

10. Kombination nach einem der Ansprüche 1 bis 9, wobei Zolmitriptan oder ein pharmazeutisch annehmbares Salz davon in einer Einheitsdosis von 0,5 bis 15 mg und Quetiapin oder ein pharmazeutisch annehmbares Salz davon in einer Einheitsdosis von 5 bis 50 mg verabreicht werden soll.

11. Kombination nach Anspruch 10, wobei Zolmitriptan oder ein pharmazeutisch annehmbares Salz davon in einer Einheitsdosis von 5 mg und Quetiapin oder ein pharmazeutisch annehmbares Salz davon in einer Einheitsdosis von 25 mg verabreicht werden soll.

12. Kombination nach einem der Ansprüche 1 bis 11, wobei das pharmazeutisch annehmbare Salz von Quetiapin Quetiapinfumarat ist.

13. Kombination nach einem der Ansprüche 1 bis 12, umfassend Zolmitriptan und Quetiapinfumarat.

14. Pharmazeutische Zusammensetzung, umfassend die Kombination nach einem der Ansprüche 1 bis 13 und gegebenenfalls einen pharmazeutischen Träger oder ein pharmazeutisch es Verdünnungsmittel.

15. Pharmazeutische Zusammensetzung nach Anspruch 14 zur simultanen, sequentiellen oder separaten Verwendung als Medikament.

16. Pharmazeutische Zusammensetzung nach Anspruch 15 zur simultanen, sequentiellen oder separaten verwendung zur Behandlung von Migräne oder verwandten Erkrankungen.

17. Kombination, umfassend eine erste pharmazeutische Zusammensetzung, umfassend Quetiapin oder ein pharmazeutisch annehmbares Salz davon und gegebenenfalls einen pharmazeutischen Träger oder ein pharmazeutisches Verdünnungsmittel, und eine zweite pharmazeutische Zusammensetzung, umfassend Zolmitriptan oder ein pharmazeutisch annehmbares Salz davon und gegebenenfalls einen pharmazeutischen Träger oder ein pharmazeutisches Verdünnungsmittel,

18. Verwendung einer Kombination nach einem der Ansprüche 1 bis 17 zur Herstellung eines Medikaments zur simultanen, sequentiellen oder separaten Verabreichung an einen Säuger zur Behandlung von Migräne oder verwandten Erkrankungen.

19. Verwendung einer Kombination nach einem der Ansprüche 1 bis 17 zur Herstellung eines Medikaments zur simultanen, sequentiellen oder separaten Verabreichung an einen Säuger zur Verringerung oder Beseitigung des Wiederauftretens von Migräne.

20. Verfahren zur Herstellung einer Kombination nach einem der Ansprüche 1 bis 16, wobei Quetiapin oder ein pharmazeutisch annehmbares Salz davon und Zolmitriptan oder ein pharmazeutisch annehmbares Salz davon in dieselbe pharmazeutische Zusammensetzung eingebracht werden.

21. Verfahren zur Herstellung einer Kombination nach einem der Ansprüche 1 bis 17, wobei Quetiapin oder ein pharmazeutisch annehmbares Salz davon und Zolmitriptan oder ein pharmazeutisch annehmbares Salz davon in unterschiedliche pharmazeutische Zusammensetzungen eingebracht werden.

22. Kit, umfassend Quetiapin oder ein pharmazeutisch annehmbares Salz davon und Zolmitriptan oder ein pharmazeutisch annehmbares Salz davon, gegebenenfalls mit Anweisungen und/oder Beschriftung zur verwendung.

23. Kit nach Anspruch 22, wobei Quetiapin oder ein pharmazeutisch annehmbares Salz davon und Zolmitriptan oder ein pharmazeutisch annehmbares Salz davon zur simultanen oder gleichzeitigen Verabreichung sind.

## Revendications

1. Combinaison contenant de la quetiapine ou un sel pharmaceutiquement acceptable de celle-ci et du zolmitriptan ou un sel pharmaceutiquement acceptable de celui-ci.

2. Combinaison selon la revendication 1 destinée à une utilisation simultanée, séquentielle ou séparée comme médicament.

3. Combinaison selon la revendication 1 destinée à une utilisation simultanée, séquentielle ou séparée pour le traitement de la migraine ou d'états apparentés.

4. Combinaison selon la revendication 1 destinée à une utilisation simultanée, séquentielle ou séparée pour réduire ou éliminer la récurrence de la migraine.

5. Combinaison selon l'une quelconque des revendications 1 à 4, dans laquelle la quetiapine ou un sel pharmaceutiquement acceptable de celle-ci doit être administré par voie orale et le zolmitriptan ou un sel pharmaceutiquement acceptable de celui-ci doit être administré par voie orale ou intranasale.

6. Combinaison selon la revendication 5 dans laquelle la quetiapine ou un sel pharmaceutiquement acceptable de celle-ci doit être administré par voie orale et le zolmitriptan ou un sel pharmaceutiquement acceptable de celui-ci doit être administré par voie orale.

7. Combinaison selon la revendication 6 dans laquelle la quetiapine ou un sel pharmaceutiquement acceptable de celle-ci doit être administré sous forme d'un comprimé et le zolmitriptan ou un sel pharmaceutiquement acceptable de celui-ci doit être administré sous forme d'un comprimé.

8. Combinaison selon l'une quelconque des revendications 1 à 7, dans laquelle le zolmitriptan ou un sel pharmaceutiquement acceptable de celui-ci doit être administré sous forme d'une formulation à fusion rapide.

9. Combinaison selon l'une quelconque des revendications 1 à 7 dans laquelle la quetiapine ou un sel pharmaceutiquement acceptable de celle-ci doit être administré sous une forme de dosage à libération contrôlée retardée ou à libération soutenue.

10. Combinaison selon l'une quelconque des revendications 1 à 9 dans laquelle le zolmitriptan ou un sel pharmaceutiquement acceptable de celui-ci doit être administré en une dose unitaire de 0,5 à 15 mg et la quetiapine ou un sel pharmaceutiquement acceptable de celle-ci doit être administré en une dose unitaire de 5 à 50 mg.

11. Combinaison selon la revendication 10 dans laquelle le zolmitriptan ou un sel pharmaceutiquement acceptable de celui-ci doit être administré en une dose unitaire de 5 mg et la quetiapine ou un sel pharmaceutiquement acceptable de celle-ci doit être administré en une dose unitaire de 25 mg.

12. Combinaison selon l'une quelconque des revendications 1 à 11 dans laquelle le sel pharmaceutiquement acceptable de la quetiapine est le fumarate de quetiapine.

13. Combinaison selon l'une quelconque des revendications 1 à 12 comprenant du zolmitriptan et du fumarate de quetiapine.

14. Préparation pharmaceutique comprenant la combinaison selon l'une quelconque des revendications 1 à 13 et éventuellement un support ou un diluant pharmaceutique.

15. Préparation pharmaceutique selon la revendication 14 destinée à une utilisation simultanée, séquentielle ou séparée comme médicament.

16. Préparation pharmaceutique selon la revendication 15 destinée à une utilisation simultanée, séquentielle ou séparée dans le traitement de la migraine ou d'états apparentés.

17. Combinaison contenant une première préparation pharmaceutique comprenant de la quetiapine ou un sel pharmaceutiquement acceptable de celle-ci et éventuellement un support ou un diluant pharmaceutique et une deuxième préparation pharmaceutique comprenant du zolmitriptan ou un sel pharmaceutiquement acceptable de celui-ci et éventuellement un support ou un diluant pharmaceutique.

18. Utilisation d'une combinaison selon l'une quelconque des revendications 1 à 17 pour la fabrication d'un médicament destiné à une administration simultanée, séquentielle ou séparée à un mammifère pour le traitement de la migraine ou d'états apparentés.

19. Utilisation d'une combinaison selon l'une quelconque des revendications 1 à 17 pour la fabrication d'un médicament destiné à une administration simultanée, séquentielle ou séparée à un mammifère pour réduire ou éliminer la récurrence de la migraine.

20. Procédé pour la préparation d'une combinaison selon l'une quelconque des revendications 1 à 16 dans lequel la quetiapine ou un sel pharmaceutiquement acceptable de celle-ci et le zolmitriptan ou un sel pharmaceutiquement acceptable de celui-ci sont incorporés dans la même préparation pharmaceutique.

21. Procédé pour la préparation d'une combinaison selon l'une quelconque des revendications 1 à 17 dans lequel la quetiapine ou un sel pharmaceutiquement acceptable de celle-ci et le zolmitriptan ou un sel pharmaceutiquement acceptable de celui-ci sont incorporés dans des préparations pharmaceutiques différentes.

22. Kit comprenant de la quetiapine ou un sel pharmaceutiquement acceptable de celle-ci et du zolmitriptan ou un sel pharmaceutiquement acceptable de celui-ci, éventuellement avec des instructions et/ou un marquage pour l'utilisation.

23. Kit selon la revendication 22 dans lequel la quetiapine ou un sel pharmaceutiquement acceptable de celle-ci et le zolmitriptan ou un sel pharmaceutiquement acceptable de celui-ci sont destinés à une administration simultanée ou contemporaine.
